# EUROPEAN PATENT SPECIFICATION

(11) **EP 4 169 463 B1**
(45) Date of publication and mention of the grant of the patent: **15.07.2026**
(21) Application number: 22210328.5
(22) Date of filing: 29.11.2022
(51) Int. Cl.: A61B 18/14, H05K 1/00

(54) **DEVICES FOR AN EXPANDABLE CATHETER ASSEMBLY**
VORRICHTUNGEN FÜR EINEN EXPANDIERBAREN KATHETER
DISPOSITIFS POUR UN CATHÉTER EXTENSIBLE

(30) Priority: 30.09.2021 US 202117489895
(43) Date of publication of application: 26.04.2023
(73) Proprietor: Biosense Webster (Israel) Ltd, 2066717 Yokneam (IL)
(72) Inventor: BEECKLER, Christopher Thomas, Irvine, 92618 (US); HERRERA, Kevin Justin, Irvine, 92618 (US); PAPAIOANNOU, Athanassios, Irvine, 92618 (US); KEYES, Joseph Thomas, Irvine, 92618 (US)
(74) Representative: Carpmaels & Ransford LLP

(56) References cited:
- US-A1- 2012 271 135
- US-A1- 2019 021 620
- US-A1- 2020 138 512
- US-A1- 2020 337 765
- US-A1- 2021 187 241

## Description

### FIELD OF THE INVENTION

The present invention relates to medical equipment, and in particular, but not exclusively, to expandable assembly catheters.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/006455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied to the tip electrode(s) of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

US Patent Publication 2013/0253298 of Harlev, et al., describes a multi electrode catheter for non-contact mapping of the heart having independent articulation and deployment features.

US Patent Publication 2012/0239028 of Wallace, et al., describes in one embodiment, a device including an expandable support member having a first portion and a second portion. The first portion is adapted to have a smaller expansion index than the second portion. A therapeutic or diagnostic instrument is supported, at least in part, by the expandable support member first portion. In another embodiment, the support member is adapted for non-uniform expansion of the first and second portions. There are also described methods of forming therapeutic devices. There are also described methods of providing therapy to tissue in a body by positioning a device in proximity to tissue in a body selected to receive therapy. Next, the expandable support member second portion is expanded until the instrument is at a therapeutic position relative to the tissue in a body selected to receive therapy. Thereafter, therapy or diagnosis is provided to the selected tissue using the device.

US Patent 5,823,189 to Kordis describes an electrode support structure has at least two spline leaves, each comprising an opposed pair of spline elements connected by a center web. Each web has a hole through which a pin assembly extends to join the webs of the spline leaves in a mutually stacked relationship. The spline elements radiate from the pin assembly in a circumferentially spaced relationship for carrying one or more electrodes. A hub member is over-molded about the pin assembly.

US Patent 8,644,902 to Kordis, et al., describes a method for sensing multiple local electric voltages from endocardial surface of a heart, and includes providing a system for sensing multiple local electric voltages from endocardial surface of a heart, including: a first elongate tubular member having a lumen, a proximal end and a distal end; a basket assembly including: a plurality of flexible splines for guiding a plurality of exposed electrodes, the splines having proximal portions, distal portions and medial portions therein between, wherein the electrodes are substantially flat electrodes and are substantially unidirectionally oriented towards a direction outside of the basket.

US 2019/0021620 A1 provides a partially-masked electrode that includes a conductive material and an insulated coating having an outer surface. The insulated coating defines a contoured opening that exposes or reveals an area of the conductive material, wherein the contoured opening has an upper perimeter at the outer surface of the insulated coating.

US 2020/0337765 A1 provides various embodiments, systems, devices and methods for modulating targeted nerve fibers ( e.g., hepatic neuromodulation) or other tissue.

US 2021/0187241 A1 provides a catheter apparatus, including an elongated deflectable element including a distal end, a coupler connected to the distal end, a pusher including a distal portion, and configured to be advanced and retracted through the deflectable element, a nose connector connected to the distal portion, and including a distal receptacle having an inner surface and a distal facing opening, and an expandable assembly including flexible polymer circuit strips, each strip including electrodes disposed thereon, the strips being disposed circumferentially around the distal portion of the pusher, with first ends of the strips being connected to the coupler and second ends of the strips including respective hinges entering the distal facing opening and connected to the inner surface of the distal receptacle, the strips being configured to bow radially outward when the pusher is retracted expanding the expandable assembly from a collapsed form to an expanded form.

US 2012/0271135 A1 provides A device for insertion into a body lumen, includes: an electrode assembly strip with exposed electrodes including: a polymeric substrate having an upper surface and an opposed lower surface; one or more electrodes disposed over a portion of the upper surface of the polymeric substrate; one or more electrical traces disposed over a portion of the lower surface of the polymeric substrate in electrical communication with the one or more electrodes by way of metal plated holes through the substrate; and a flexible polymeric substrate having a substrate surface and a substrate wall; wherein the electrode assembly strip is compressingly and thermally bonded to the substrate surface of the flexible polymeric substrate to define a flexible electrode assembly strip; and wherein the electrode assembly strip has a thickness from about 0.0005 inches to about 0.008 inches.

### SUMMARY OF THE INVENTION

The invention is defined in claim 1. Advantageous embodiments are defined in the dependent claims.

### SUMMARY OF THE DISCLOSURE

There is provided in accordance with an embodiment of the present disclosure, a catheter apparatus, including an elongated deflectable element including a distal end, a coupler connected to the distal end, a pusher including a distal portion, and being configured to be advanced and retracted through the deflectable element, a nose connector connected to the distal portion of the pusher, and including a distal receptacle having an inner surface and a distal facing opening, and an expandable assembly including a plurality of flexible polymer circuit strips, each flexible polymer circuit strip including multiple electrodes disposed thereon, the flexible polymer circuit strips being disposed circumferentially around the distal portion of the pusher, with first ends of the strips being connected to the coupler and second ends of the strips including respective hinges entering the distal facing opening and connected to the inner surface of the distal receptacle of the nose connector, the strips being configured to bow radially outward when the pusher is retracted expanding the expandable assembly from a collapsed form to an expanded form.

Further in accordance with an embodiment of the present disclosure the respective hinges are configured to provide a maximum angular range of movement, which is in excess of 80 degrees, between the collapsed form and the expanded form.

Still further in accordance with an embodiment of the present disclosure the hinges have a thickness in the range of 10 to 140 microns.

Additionally, in accordance with an embodiment of the present disclosure, the apparatus includes respective elongated resilient support elements connected along a given length of respective ones of the flexible polymer circuit strips providing a shape of the expandable assembly in the expanded form.

Moreover, in accordance with an embodiment of the present disclosure the elongated resilient support elements include Nitinol.

Further in accordance with an embodiment of the present disclosure the elongated resilient support elements include Polyetherimide (PEI).

Still further in accordance with an embodiment of the present disclosure the respective elongated resilient support elements extend along the respective strips from the coupler until before the respective hinges.

Additionally, in accordance with an embodiment of the present disclosure the flexible polymer circuit strips include a polyimide layer.

Moreover, in accordance with an embodiment of the present disclosure the hinges of the flexible polymer circuit strips are supported with a length of yarn.

Further in accordance with an embodiment of the present disclosure the yarn includes any one or more of the following an ultra-high-molecular-weight polyethylene yarn, or a yarn spun from a liquid-crystal polymer.

Still further in accordance with an embodiment of the present disclosure the flexible polymer circuit strips are covered with a thermoplastic polymer resin shrink wrap (PET).

Additionally, in accordance with an embodiment of the present disclosure respective ones of the second ends of respective ones of the flexible polymer circuit strips are tapered along the width of the respective ones of the flexible polymer circuit strips.

Moreover, in accordance with an embodiment of the present disclosure the coupler has an inner surface, the first ends of the strips being connected to the inner surface of the coupler.

Further in accordance with an embodiment of the present disclosure respective ones of the first ends of respective ones of the flexible polymer circuit strips include an electrical connection array.

Still further in accordance with an embodiment of the present disclosure, the apparatus includes a position sensor disposed in the distal receptacle of the nose connector.

Additionally, in accordance with an embodiment of the present disclosure, the apparatus includes a position sensor disposed between the coupler and the pusher.

Moreover, in accordance with an embodiment of the present disclosure, the apparatus includes a nose cap covering the distal facing opening of the nose connector.

Further, in accordance with an embodiment of the present disclosure, the catheter apparatus includes a covering that can at least partially enclose the flexible polymer circuit strip and the multiple electrodes.

Still further, in accordance with an embodiment of the present disclosure, the covering includes a plurality of apertures at each electrode of the multiple electrodes so that a portion of the conductive surface of each electrode is exposed through each aperture of the plurality of apertures.

Additionally, in accordance with an embodiment of the present disclosure, the covering includes a non-conductive polymer material.

Moreover, in accordance with an embodiment of the present disclosure, the conductive surface of each electrode is disposed approximately 12 microns below an outer surface of the covering.

Further, in accordance with an embodiment of the present disclosure, the catheter apparatus includes a conductive polymer coating disposed in each aperture of the plurality of apertures such that input impedance to each electrode measures at less than 13,000 ohms at 1 Hz.

Still further, in accordance with an embodiment of the present disclosure, the plurality of apertures include a plurality of circular apertures, polygonal apertures (e.g., rectangular, triangular, or decagonal apertures), or elongated slits at each electrode of the multiple electrodes.

Additionally, in accordance with an embodiment of the present disclosure, the elongated slits extend from near a first end of the electrode to near a second end of the electrode.

Moreover, in accordance with an embodiment of the present disclosure, the disclosed technology include a flexible polymer circuit strip for a catheter.

Further, in accordance with an embodiment of the present disclosure, the flexible polymer circuit strip includes an elongated resilient support element and a flexible polymer circuit connected to the elongated resilient support element and a flexible polymer circuit.

Still further, in accordance with an embodiment of the present disclosure, the flexible polymer circuit includes a plurality of electrodes with each electrode defining a first conductive surface area.

Additionally, in accordance with an embodiment of the present disclosure, the flexible polymer circuit strip includes a covering that at least partially encloses the elongated resilient support element, the flexible polymer circuit and the plurality of electrodes.

Moreover, in accordance with an embodiment of the present disclosure, the covering includes a plurality of apertures over each electrode of the plurality of electrodes so that the apertures over each electrode collectively defines a second conductive surface area of approximately less than half of the first conductive surface area.

Further, in accordance with an embodiment of the present disclosure, the disclosed technology includes a method of manufacturing a flexible polymer circuit strip for a catheter.

Still further, in accordance with an embodiment of the present disclosure, the method includes placing an elongated resilient support element, a flexible polymer circuit comprising a plurality of electrodes, and a yarn together into a thermoplastic polymer resin shrink wrap such that the thermoplastic polymer resin shrink wrap covers the plurality of electrodes of the flexible polymer circuit.

Additionally, in accordance with an embodiment of the present disclosure, the method includes heating the thermoplastic polymer resin shrink wrap to cause the thermoplastic polymer resin shrink wrap to shrink and at least partially enclose the elongated resilient support element, the flexible polymer circuit, and the yarn.

Moreover, in accordance with an embodiment of the present disclosure, the method includes forming a plurality of apertures through the thermoplastic polymer resin shrink wrap at each electrode of the plurality of electrodes.

Further, in accordance with an embodiment of the present disclosure, the step of forming the plurality of apertures through the thermoplastic polymer resin shrink wrap includes cutting the plurality of apertures through the thermoplastic polymer resin shrink wrap with a laser.

Still further, in accordance with an embodiment of the present disclosure, the step of forming the plurality of apertures through the thermoplastic resin shrink wrap with the laser includes cutting a plurality of circular apertures through the thermoplastic resin shrink wrap with the laser.

Further, in accordance with an embodiment of the present disclosure, a flexible electrode device can comprise a flexible polymer circuit strip and at least two electrodes disposed on the flexible polymer circuit strip. The flexible electrode device can further include a covering partially enclosing the flexible polymer circuit strip and the at least two electrodes. The covering can include a plurality of apertures at each electrode of the at least two electrodes. The flexible electrode device can further include a conductive polymer disposed in each of the plurality of apertures so that an impedance measured from the electrodes is less than 13,000 ohms at 1 Hz.

Further, in accordance with an embodiment of the present disclosure, an impedance measured from the electrodes can be less than 1400 ohms at 10 Hz, approximately 300 ohms or less at 50 Hz, and approximately 200 ohms or less at 100 Hz. Furthermore, the plurality of apertures can include two rows of five substantially circular apertures in each row. In another embodiment of the present disclosure, the plurality of apertures can be three rows of seven substantially circular apertures in each row.

### BRIEF DESCRIPTION OF THE DRAWINGS

The present invention will be understood from the following detailed description, taken in conjunction with the drawings in which:
Fig. 1 is a schematic view of a basket catheter constructed and operative in accordance with an embodiment of the present invention;
Figs. 2 and 3 are more detailed views of the expandable assembly of the basket catheter of Fig. 1;
Fig. 4 is a partly exploded view of the basket catheter of Fig. 1;
Fig. 5 is an enlarged view of a nose section of the basket catheter of Fig. 1 with a nose cap removed;
Figs. 6A and 6B are schematic views of the expandable assembly of the basket catheter of Fig. 1 in expanded and collapsed form;
Fig. 7 is a schematic view of the flexible polymer circuit strips for use in the basket catheter of Fig. 1;
Fig. 8A is a cross-sectional view through line A-A of Fig. 7;
Figs. 8B-8I illustrate example apertures formed in a covering of the flexible polymer circuit strips of Fig. 7;
Fig. 8J is a table illustrating impedance values for example aperture patterns formed in the covering of the flexible polymer circuit strips of Fig. 7;
Fig. 9 is a schematic view of a deflectable element of the basket catheter of Fig. 1;
Fig. 10 is a schematic view of an irrigation sleeve of the basket catheter of Fig. 1;
Fig. 11 is a schematic view of a pusher of the basket catheter of Fig. 1;
Fig. 12 is a schematic view of a multi-axis position sensor of the basket catheter of Fig. 1;
Figs. 13A-B are schematic views of a nose connector of the basket catheter of Fig. 1;
Fig. 14 is a schematic view of a nose connector retainer of the basket catheter of Fig. 1;
Figs. 15A-B are schematic views of a nose cap of the basket catheter of Fig 1;
Fig. 16 is a schematic view of a coupler of the basket catheter of Fig. 1;
Fig. 17 is a schematic view of a single-axis position sensor of the basket catheter of Fig. 1;
Fig. 18 is a schematic view of a proximal retainer ring of the basket container of Fig. 1;
Figs. 19-20 are cross sectional views through line A-A of Fig. 1; and
Fig. 21 illustrates a flowchart of a method of forming a flexible polymer circuit strip of the basket catheter of Fig. 1 in accordance with an embodiment of the present invention.

### DESCRIPTION OF EXAMPLE EMBODIMENTS

### OVERVIEW

Investigative electrodes on basket catheters are generally distributed along the length of the splines of the basket assembly. Proximal ends of the splines of the basket assembly are generally connected to an insertion tube of the catheter, while distal ends of the splines are connected to a pusher which is disposed within an insertion tube. The pusher may be retracted and advanced, to expand and collapse, the basket assembly, respectively. When the basket assembly is collapsed, the splines have a substantially linear formation, with the distal ends of the splines being connected to outer surface of the pusher and typically covered with a cap forming the nose of the catheter. When the basket assembly is expanded the nose of the catheter protrudes distally beyond the expanded assembly.

During investigative procedures, the tissue region contacted by the distal portion of the basket is of greater interest than other regions for investigative purposes, but due to the nose of the basket protruding beyond the expanded assembly, some of the distal portion surrounding the nose of the basket assembly is prevented from making contact with tissue thereby preventing using some of that distal portion for investigative purposes.

Basket catheters with flatter noses have been proposed, but generally these catheters suffer from various disadvantages such as the nose is not flat enough, the basket does not collapse sufficiently, and/or the structural engineering of the basket is deficient in one or more ways such that the basket fails under compression and/or tension when being deployed and/or in use.

Embodiments of the present invention solve the above problems by providing a catheter apparatus including an expandable basket assembly with a substantially flat nose so that electrodes may be placed close to the nose and still make contact with tissue when the basket assembly is expanded. The distal ends of the splines include hinges which are flexible enough and have a large enough angular range of bending to allow the expandable assembly to achieve its fully expanded form and its fully collapsed form, while being strong enough to withstand the various compressive and tensile stresses applied to the catheter. The distal ends of the splines are tucked into, and connected to, a receptacle at the end of the pusher so that the end of the catheter is either level with the basket assembly when the basket is expanded or only sticks out at minimal distance (for example, up to about 1 mm) from the expanded basket assembly.

In some embodiments, the catheter apparatus includes an elongated deflectable element, a coupler connected to the distal end of the deflectable element, and a pusher, which may be advanced and retracted through the deflectable element. The apparatus also includes a nose connector connected to the distal portion of the pusher, and an expandable assembly comprising flexible polymer circuit strips. Each flexible polymer circuit strip includes multiple electrodes disposed thereon. The flexible polymer circuit strips are placed circumferentially around the distal portion of the pusher, with first ends of the strips being connected to the coupler and second ends of the strips comprising respective hinges entering a distal facing opening of a distal receptacle of the nose connector and connected to the inner surface of the distal receptacle of the nose connector. The strips are configured to bow radially outward when the pusher is retracted expanding the expandable assembly from a collapsed form to an expanded form.

In some embodiments, the second ends of the flexible polymer circuit strips are tapered along their width to facilitate insertion of the strips into the receptacle without overlap. In some embodiments, the first ends of the strips are connected to the inner surface of the coupler.

The apparatus includes respective elongated resilient support elements connected along a given length of respective ones of the flexible polymer circuit strips providing a shape of the expandable assembly in the expanded form. The respective elongated resilient support elements extend along the respective strips from the coupler until before the respective hinges thereby providing the strips with sufficient resilience where needed without adding bulk to the hinges. The elongated resilient support elements may include any suitable resilient material, for example, but not limited to, Nitinol and/or Polyetherimide (PEI).

The flexible polymer circuit strips may include a polyimide layer. The hinges of the flexible polymer circuit strips may be strengthened with any suitable material, for example, but not limited to, a length of yarn, which is flexible and provides tensile support to the strips. In some embodiments, a length of yarn runs the whole length of each strip including the hinges. The yarn may include any suitable yarn. For example, the yarn may include one or more of the following: an ultra-high-molecular-weight polyethylene yarn; or a yarn spun from a liquid-crystal polymer. Each flexible polymer circuit strip, its length of yarn, and elongated resilient support element may be secured together with a suitable adhesive, for example, epoxy, and then covered with a thermoplastic polymer resin shrink wrap (PET) or any other suitable covering. Windows may be created in the PET covering with a laser, mechanical removal, or any other suitable method in order to expose the electrodes. Alternatively, prior to shrinking, the PET covering may already have windows present.

The flexible polymer circuit strips may further include a conductive polymer coating, such as poly(3,4-ethylenedioxythiophen) (PEDOT) or poly(3, 4 ethylenedioxythiophene) polystyrene sulfonate (PEDOT:PSS), over each electrode to help protect the electrode, reduce input impedance, and enhance the signal-to-noise ratio. The conductive polymer coating may be applied to each electrode by dipping the electrode in a solution comprising the conductive polymer coating and then passing an electrical current through the electrode. As the current passes through each electrode, the conductive polymer coating adheres to the surface of the electrode.

To help reduce the likelihood that the conductive polymer coating is damaged by rubbing on the sheath or contacting other objects, the disclosed technology can include forming apertures in the PET covering with a laser, mechanical removal, or any other suitable method in order to expose only a portion of each electrode. In other words, rather than removing the PET covering to expose the entire surface of the electrode, the disclosed technology can include removing smaller portions of the PET covering to form small apertures through the PET covering to expose only portions of the electrode's surface. By including small apertures through the PET, the PET can provide protection to the conductive polymer coating which is positioned in each aperture by preventing the conductive polymer coating from contacting the sheath or other objects. The apertures can be sized, shaped, and positioned to help reduce the likelihood that the conductive polymer coating will contact the sheath or other objects while also ensuring the electrode is capable of detecting electrical signals of the heart. Reduction in damage to the conductive polymer coating may result in more accurate signals from the electrodes and/or less risk of a health threat due to shedding of coating into the patient's heart and/or vasculature.

In some embodiments, each flexible polymer circuit strip may be electrically isolated from its elongated resilient support element, for example, by coating the elongated resilient support element with an insulator or by using a covering such as a shrink wrap which wraps the elongated resilient support element and the length of yarn. In some embodiments, the elongated resilient support elements may be non-conductive.

The hinges (including the yarn and covering layers) may have any suitable thickness, for example, in the range of 10 to 140 microns.

The catheter apparatus may include one or more positions sensors, for example, a position sensor (e.g., a multi-axis sensor) disposed in the distal receptacle of the nose connector, and/or a position sensor (e.g., a single-axis sensor) disposed between the coupler and the pusher. A nose cap may be used to cover the distal facing opening of the nose connector.

### SYSTEM DESCRIPTION

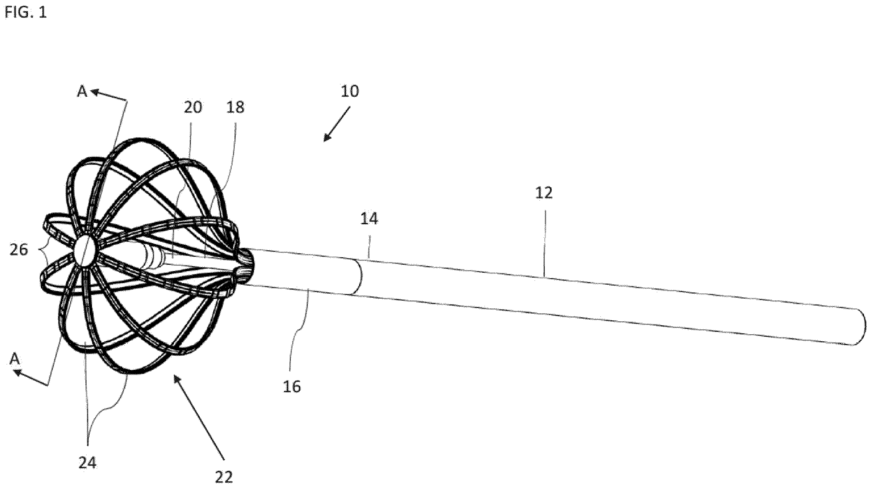

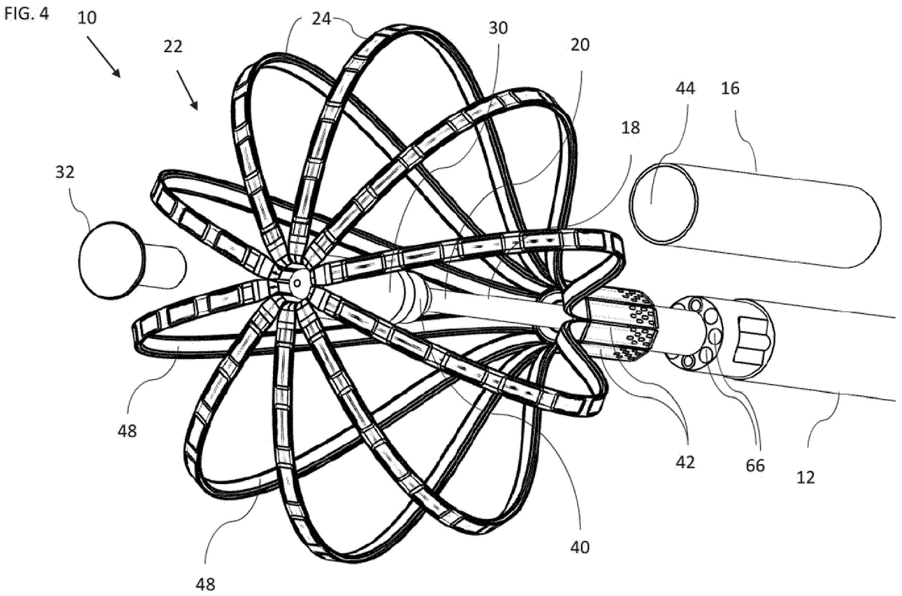

Reference is now made to Fig. 1, which is a schematic view of a basket catheter 10 constructed and operative in accordance with an embodiment of the present invention. The basket catheter 10 includes an elongated deflectable element 12 having a distal end 14, a coupler 16 connected to the distal end 14, and a pusher 18 including a distal portion 20. The pusher 18 is configured to be advanced and retracted through the deflectable element 12, for example, using a manipulator or handle (not shown). The basket catheter 10 also includes an expandable assembly 22 comprising a plurality of flexible polymer circuit strips 24 (only some labeled for the sake of simplicity). Each flexible polymer circuit strip 24 includes multiple electrodes 26 disposed thereon (only some labeled for the sake of simplicity). The formation of the various elements and how they are connected with each other are described in more detail with reference to the Figs. 4-20.

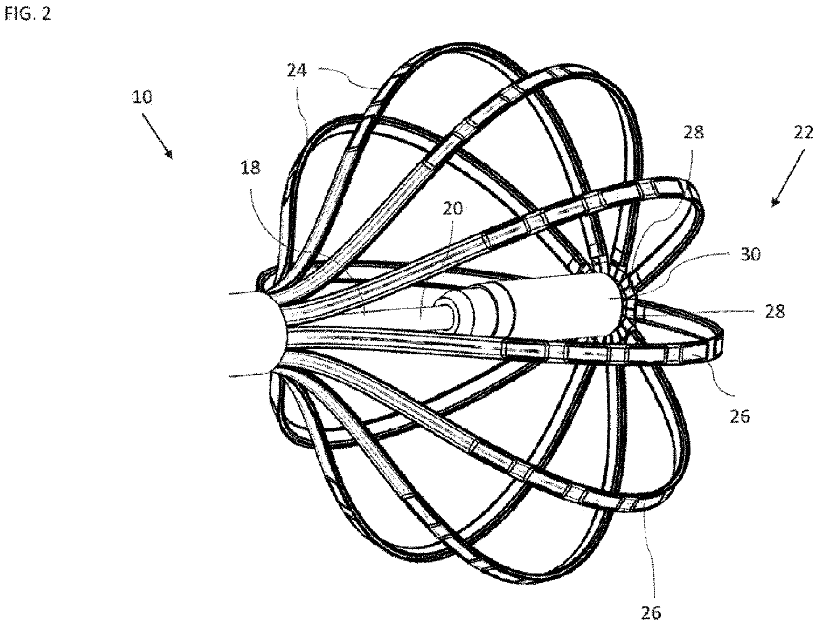

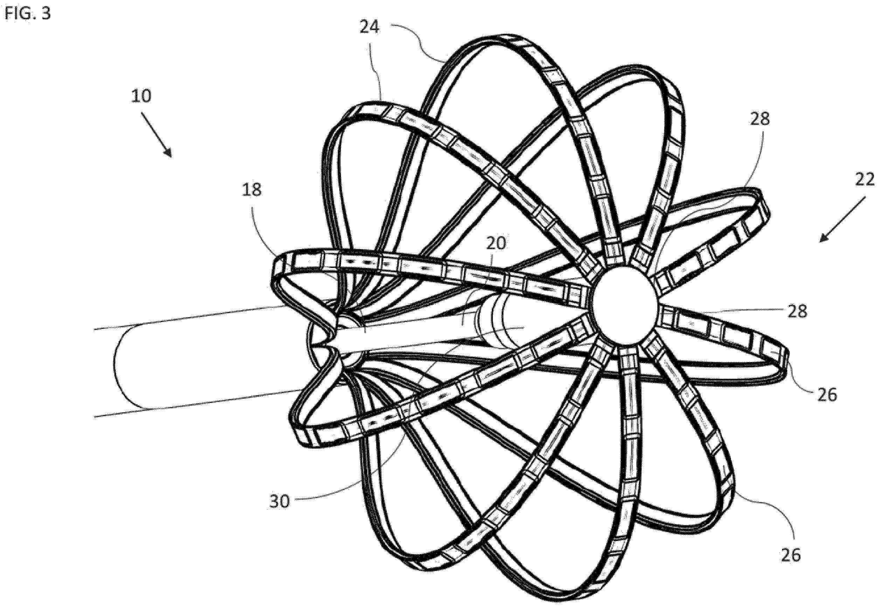

Reference is now made to Figs. 2 and 3, which are more detailed views of the expandable assembly 22 of the basket catheter 10 of Fig. 1. Figs. 2 and 3 show the electrodes 26 on the flexible polymer circuit strips 24 more clearly. Fig. 2 shows that the electrodes 26 are not disposed on the proximal portions of the flexible polymer circuit strips 24. The basket catheter 10 includes a nose connector 30 connected to the distal portion 20 of the pusher 18. The flexible polymer circuit strips 24 are connected via hinges 28 (only some labeled for the sake of simplicity) of the flexible polymer circuit strips 24 to the nose connector 30.

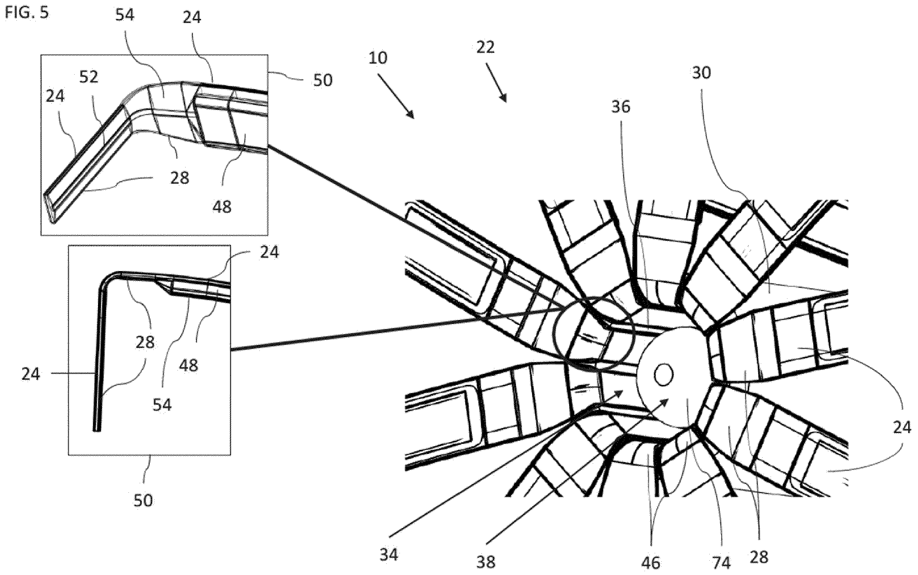

Reference is now made to Figs. 4-5. Fig. 4 is a partly exploded view of the basket catheter 10 of Fig. 1. Fig. 5 is an enlarged view of a nose section of the basket catheter 10 of Fig. 1 with a nose cap 32 removed.

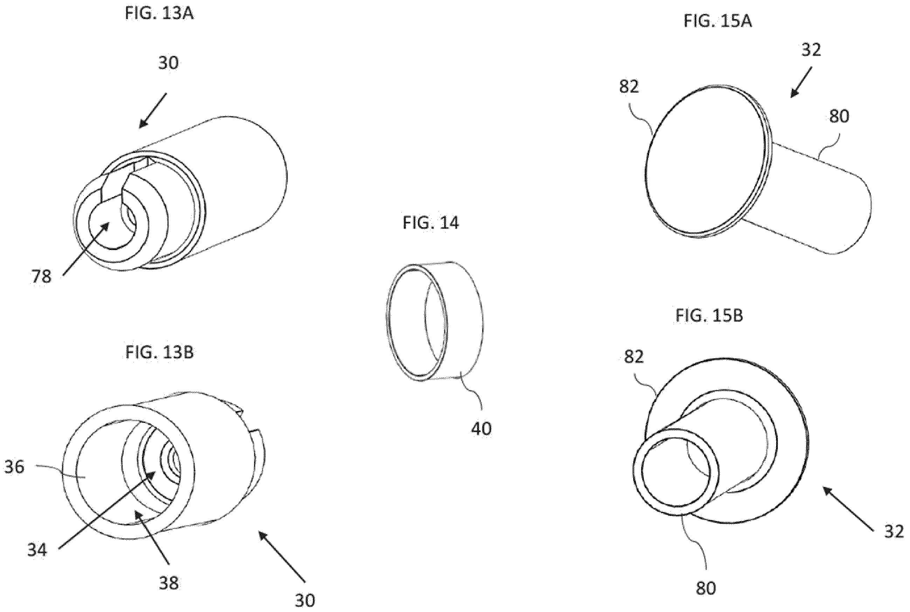

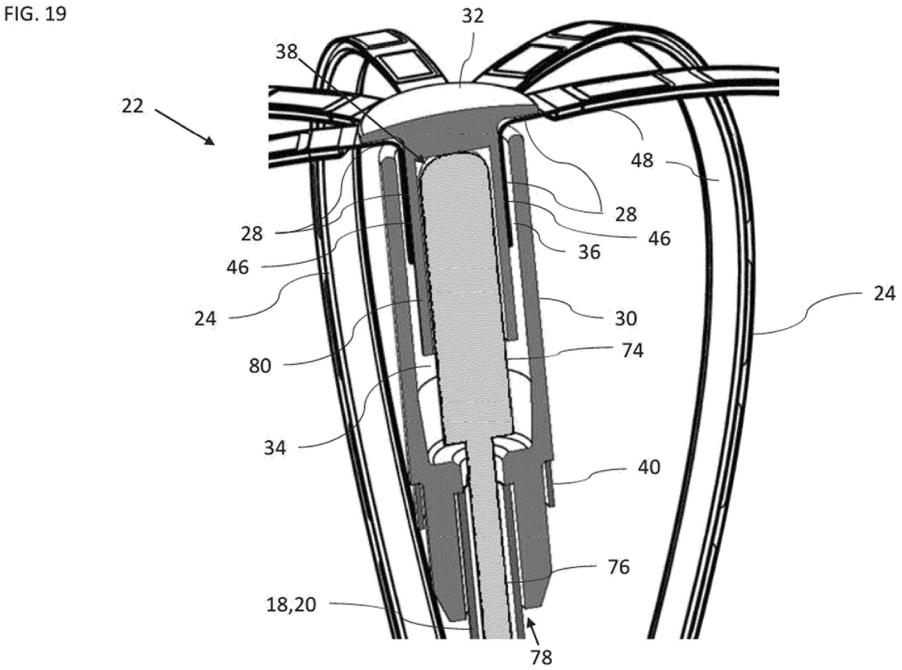

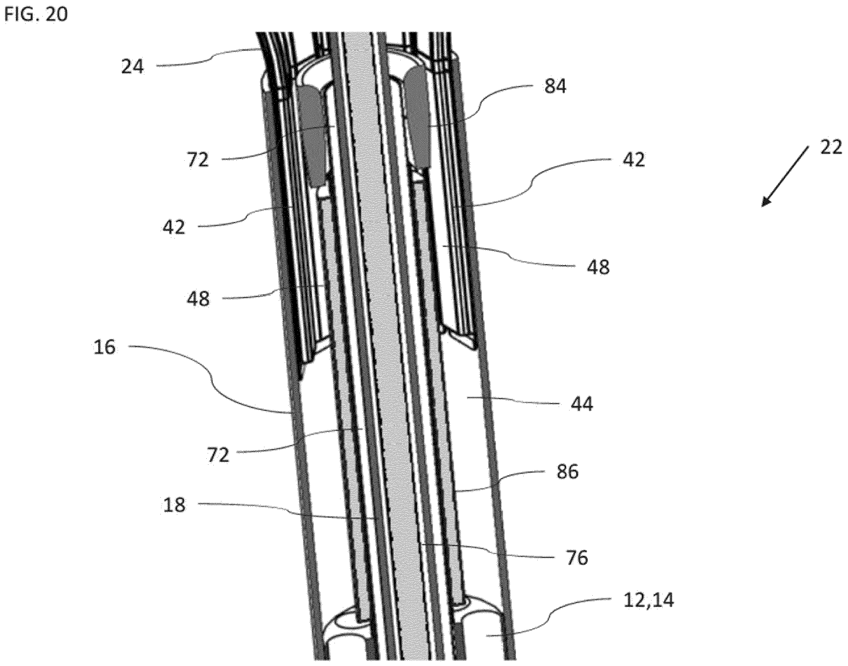

Fig. 4 shows the nose cap 32 and the coupler 16 removed from the basket catheter 10 to illustrate how the flexible polymer circuit strips 24 are connected to the nose connector 30 and the coupler 16. The nose connector 30 is connected to the distal portion 20 of the pusher 18. The proximal end of the coupler 16 may be connected to the elongated deflectable element 12 using any suitable connection method, such as using adhesive, for example, epoxy. The nose connector 30 is secured to the distal portion 20 of the pusher 18 using a center electrode ring 40, which is described in more detail with reference to Figs. 14 and 19. The flexible polymer circuit strips 24 are disposed circumferentially around the distal portion 20 of the pusher 18, with first ends 42 (only some labeled for the sake of simplicity) of the strips 24 being connected to an inner surface 44 of the coupler 16. The connection between the flexible polymer circuit strips 24 and the inner surface 44 is shown more clearly with reference to Fig. 20.

Fig. 5 shows that the nose connector 30 includes a distal receptacle 34 having an inner surface 36 and a distal facing opening 38. The nose connector 30 is described in more detail with reference to Figs. 13A-B and 19. Fig. 5 shows that second ends 46 (Fig. 5) (only some labeled for the sake of simplicity) of the strips 24 comprising the respective hinges 28 (Fig. 5) entering the distal facing opening 38 (Fig. 5) and are connected to the inner surface 36 (Fig. 5) of the distal receptacle 34 (Fig. 5) of the nose connector 30.

Fig. 4 shows that the basket catheter 10 also includes respective elongated resilient support elements 48 connected along a given length of respective ones of the flexible polymer circuit strips 24 providing a shape of the expandable assembly 22 in the expanded form of the expandable assembly 22. The elongated resilient support elements 48 may include any suitable material, for example, but not limited to, Nitinol and/or Polyetherimide (PEI).

Fig. 4 shows that the respective elongated resilient support elements 48 extend along inner surface of the respective strips 24 from the coupler 16, while Fig. 5 shows that the elongated resilient support elements 48 extend along the respective flexible polymer circuit strips 24 until before the respective hinges 28. Insets 50 of Fig. 5 show one of the hinges 28 and a portion of one of the flexible polymer circuit strips 24 adjacent to that hinge 28. The insets 50 illustrate that the elongated resilient support element 48 does not extend to the region of the hinge 28. It can also be seen that the hinge region is much thinner than the region including the elongated resilient support element 48. The hinges 28 may have any suitable thickness, for example, in the range of approximately 10 to approximately 140 microns. The strip 24 are folded such that strip 24 defines a generally perpendicular configuration (inset 50) to each other.

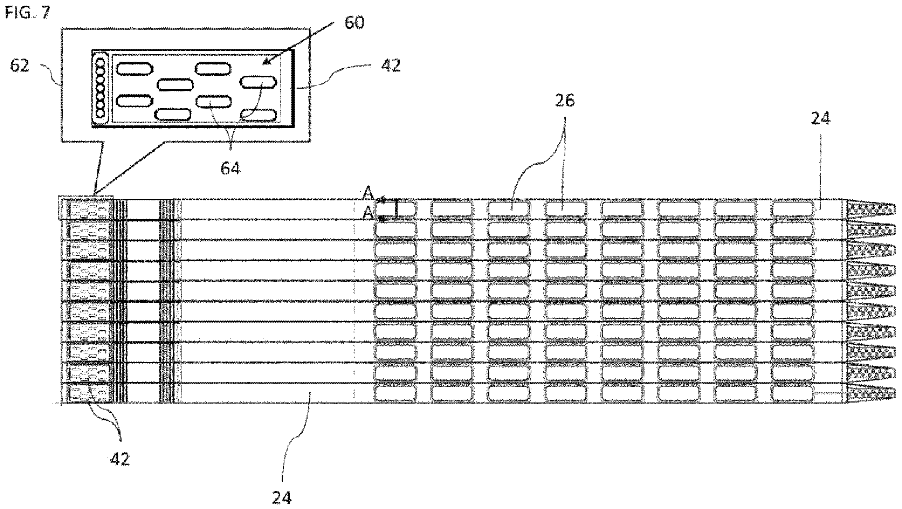

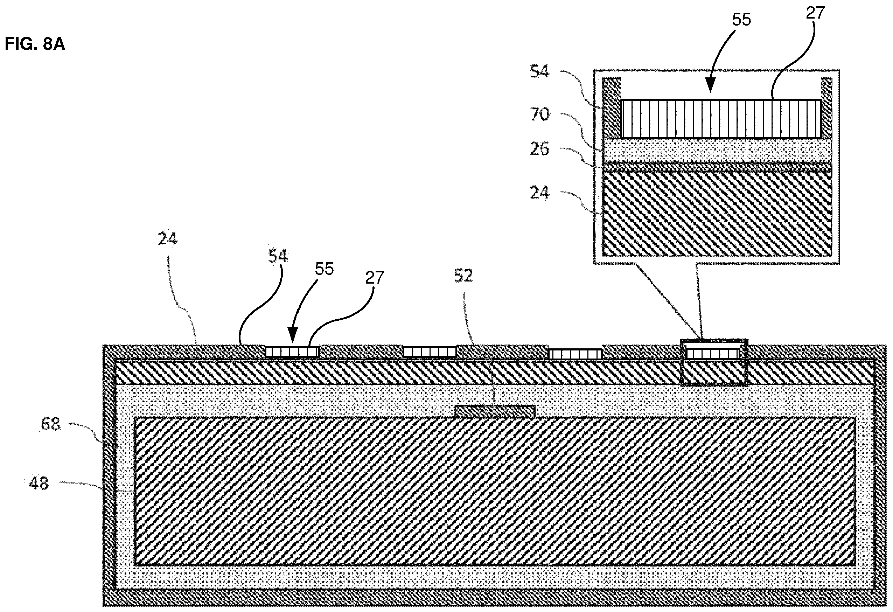

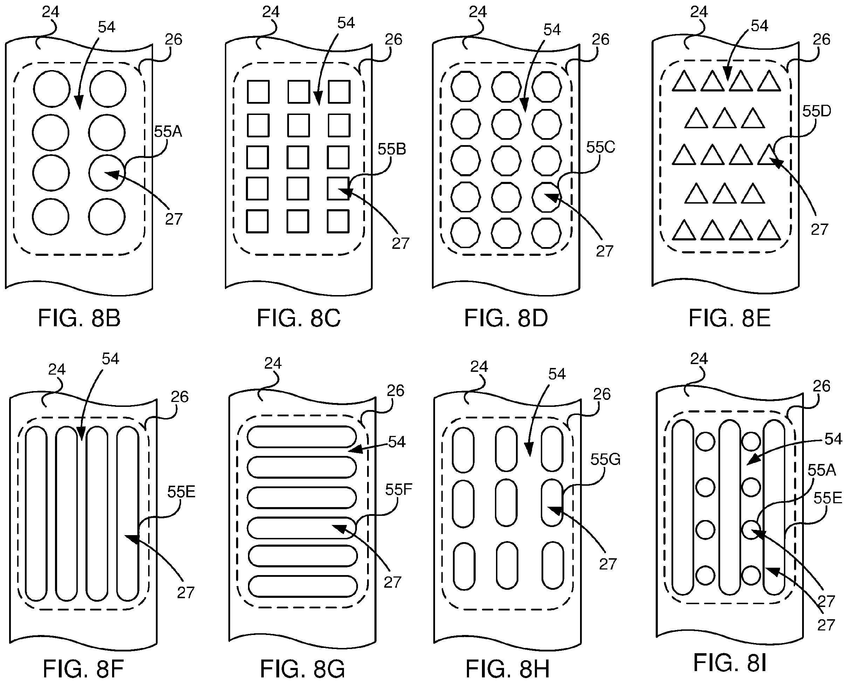

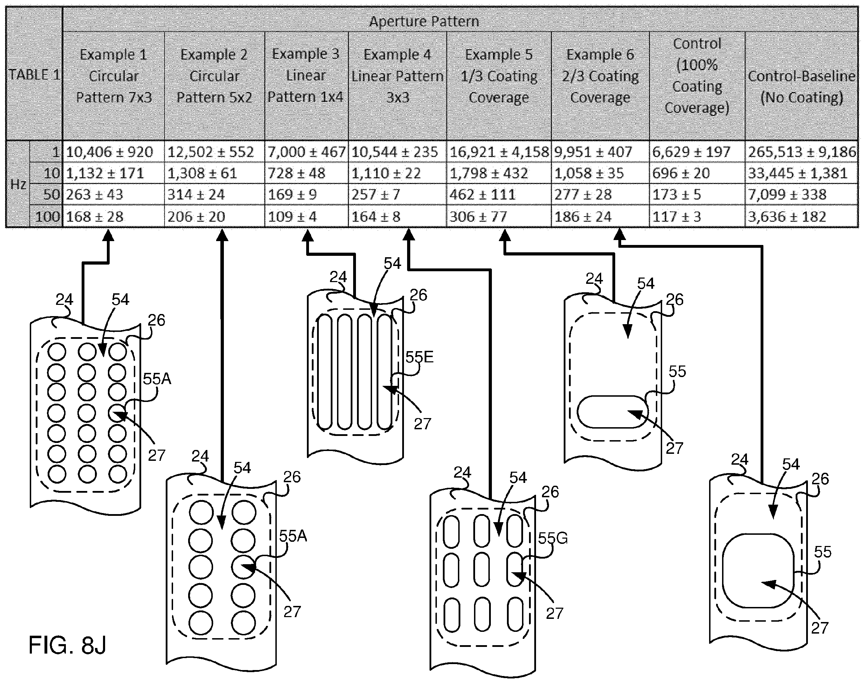

In some embodiments, each of the flexible polymer circuit strips 24 comprises a polyimide layer. The flexible polymer circuit strips 24 may be composed of any suitable materials. The flexible polymer circuit strips 24 are described in more detail with reference to Figs. 7 and 8.

Fig. 5 also shows that respective ones of the second ends 46 of respective ones of the flexible polymer circuit strips 24 are tapered along the width of the respective ones of the flexible polymer circuit strips 24 to allow inserting the second ends 46 into the distal receptacle 34 without overlap. The hinges 28 may be connected to the inner surface 36 of the distal receptacle 34 using any suitable adhesive, for example, epoxy, and/or using any suitable connection method.

The hinges 28 of the flexible polymer circuit strips 24 are supported with a length of yarn 52, which typically runs the length of each respective flexible polymer circuit strip 24. Each flexible polymer circuit strip 24 along with the yarn 52 and the associated elongated resilient support element 48 may be covered with a suitable covering 54, e.g., thermoplastic polymer resin shrink wrap (PET) described in more detail with reference to Fig. 8A. Yarn 52 can be any suitable high strength polymer including, for example, ultra high molecular weight polyethylene (Spectra or Dyneema), Kevlar, liquid crystal polymer (Vectran) and the like.

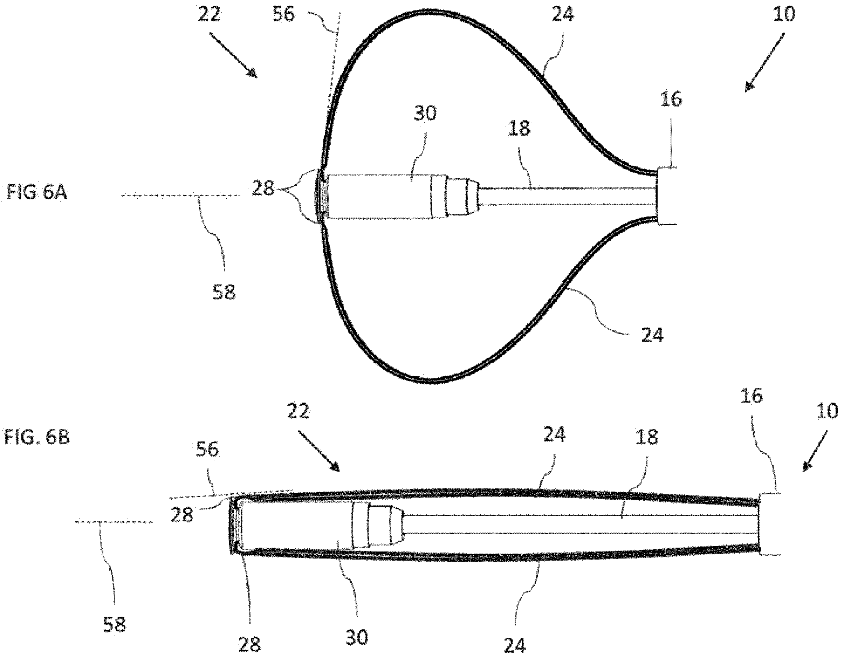

Reference is now made to Figs. 6A and 6B, which are schematic views of the expandable assembly 22 of the basket catheter 10 of Fig. 1 in expanded and collapsed form, respectively. The flexible polymer circuit strips 24 are configured to bow radially outward when the pusher 18 is retracted expanding the expandable assembly 22 from a collapsed form to an expanded form. The collapsed form of the expandable assembly 22 represents the non-stressed form of the flexible polymer circuit strips 24 which are provided with their shape using the elongated resilient support elements 48 (Fig. 4).

In some embodiments, the flexible polymer circuit strips 24 are formed as flat strips as described in more detail with reference to Fig. 7. The distal ends of the flexible polymer circuit strips 24 are connected to the inner surface 36 (Fig. 5) of the nose connector 30. At that point the flat flexible polymer circuit strips 24 are generally parallel with a line 58, which is an extension of an axis of the nose connector 30 extended distally beyond the distal end of the nose connector 30. The proximal ends of the flexible polymer circuit strips 24 are then connected to the coupler 16 so that in the collapsed form, the angle between a tangent 56 to the flexible polymer circuit strips 24 and the line 58 is close to 180 degrees, while in the expanded form, the angle between the tangent 56 and the line 58 is about 90 degrees. Therefore, in operation (when the flexible polymer circuit strips 24 are connected to the nose connector 30 and the coupler 16) the hinges 28 are configured to provide a maximum angular range of movement of the flexible polymer circuit strips 24 of about 90 degrees and generally in excess of 80 degrees. However, the hinges 28 are capable of bending 180 degrees or more. The maximum angular range is defined as the maximum angular range between the tangent 56 to the flexible polymer circuit strips 24 and the line 58. The tangent 56 to the most distal portion of the flexible polymer circuit strips 24 generally provides the maximum angular range between the flexible polymer circuit strips 24 and the line 58.

Reference is now made to Fig. 7, which is a schematic view of the flexible polymer circuit strips 24 for use in the basket catheter 10 of Fig. 1. The flexible polymer circuit strips 24 may be formed from a single piece of polymer, such as polyimide. Circuit strips 24 may be connected to each other by polyimide, or assembled as individual pieces that are held in proper alignment and secured to coupler 16. By manufacturing circuit strips 24 as individual components the yield of the base circuit may be increased as a failed electrode scraps one circuit strip rather than an entire assembly of strips. Respective first ends 42 of the respective flexible polymer circuit strips 24 include an electrical connection array 60. An inset 62 shows that the electrical connection array 60 includes electrical contacts 64 thereon (only some labeled for the sake of simplicity). The electrical contacts 64 are connected via traces (not shown) on the back of the flexible polymer circuit strips 24 to respective ones of the electrodes 26 disposed on the front of the flexible polymer circuit strips 24. Away from the region of the first ends 42, the flexible polymer circuit strips 24 are separate from each other to allow the flexible polymer circuit strips 24 to form the expandable assembly 22 (Fig. 1) when connected to the basket catheter 10. Wires (not shown) may connect the electrodes 26 to control circuitry (not shown) via the electrical contacts 64. The wires may be disposed in lumens 66 (Fig. 4) of the elongated deflectable element 12 (Fig. 4).

The flexible polymer circuit strips 24 may have any suitable dimensions. For example, the length of the flexible polymer circuit strips 24 may be in the range of 10mm to 60mm, e.g., 30 mm the width of the flexible polymer circuit strips 24 may be in the range of 0.25mm to 3mm, e.g., 0.72 mm, the thickness of the flexible polymer circuit strips 24 may be in the range of 0.005 mm to 0.14mm.

Reference is now made to Fig. 8A, which is a cross-sectional view through line A-A of Fig. 7. The yarn 52 is run along the length of the elongated resilient support element 48, e.g., formed from Nitinol or PEI, and beyond so that the yarn 52 will also run the length of the hinge 28 comprised of the flexible polymer circuit strips 24. The elongated resilient support elements 48 may have any suitable thickness, for example, in the range of 0.025 mm to 0.25 mm. A covering 68, such as a thermoplastic polymer resin shrink wrap (PET), is placed over the yarn 52 and the elongated resilient support element 48. Epoxy is injected into the covering 68. Heat is then applied to the covering thereby shrinking the covering over the yarn 52 and the elongated resilient support element 48. One reason to cover the elongated resilient support element 48 with the covering 68 is to electrically isolate the elongated resilient support element 48 from the circuit traces of the flexible polymer circuit strip 24. The covering 68 may be omitted, for example, if the elongated resilient support element 48 is covered with an insulating coating (e.g., polyurethane) or is comprised of an insulating material.

The yarn 52 may comprise any one or more of the following: an ultra-high-molecular-weight polyethylene yarn; or a yarn spun from a liquid-crystal polymer. The yarn 52 may be any suitable linear density, for example, in a range between 25 denier and 250 denier.

The flexible polymer circuit strip 24 are then placed over the yarn 52 and the elongated resilient support element 48 with the circuit trace side of the flexible polymer circuit strip 24 facing the elongated resilient support element 48 and the electrodes 26 of the flexible polymer circuit strips 24 facing away from the elongated resilient support element 48. The covering 54 is disposed around the flexible polymer circuit strip 24, yarn 52, and elongated resilient support element 48 combination, and epoxy 70 is injected into the covering 54. The covering 54 is then heated thereby shrinking the covering 54 around the combination. The flexible polymer circuit strips 24 are therefore covered with the covering 54, e.g., a thermoplastic polymer resin shrink wrap (PET).

As illustrated in Fig. 8A, apertures 55 can be formed through the covering 54 to expose the electrode 26. In some examples, the apertures 55 can expose the entire outer surface of each electrode 26 or the apertures can expose only a portion of the outer surface of each electrode 26. The apertures 55 can be formed by using a laser to cut, or otherwise remove, the covering 54 to expose the electrode 26. In other examples, the apertures 55 can be formed by mechanically removing the covering 54, by chemically etching the covering, plasma etching the covering, or by other suitable methods of removing the covering 54 to form the apertures 55. The covering 54 can be removed such that the conductive surface of each electrode 26 is disposed approximately 12 microns below an outer surface of the covering 54. As will be described in greater detail in relation to Figs. 8B-8I, if the apertures 55 expose only a portion of the outer surface of each electrode 26, the apertures 55 can comprise several small apertures 55 which collectively define a conductive area that is less than 50% of the conductive surface of the electrode 26.

Some or all of the electrodes 26 can also be coated with a coating 27 to help ensure the electrode 26 is able to properly detect electrical signals of the heart. The coating 27 can be any type of coating suitable for the application. As a non-limiting example, the coating 27 can be poly(3,4-ethylenedioxythiophene) (PEDOT), poly(3, 4 ethylenedioxythiophene) polystyrene sulfonate (PEDOT:PSS), electrochemically grown iridium oxide, electrochemically grown Titanium Nitride (TiN) or any other suitable coating for the particular application. The coating 27 can help to reduce the overall impedance of the electrode 26. In some examples, the coating 27 can be applied to the exposed surface of the electrode 26 such that the overall impedance can be reduced by about 99% at low frequencies. As an example, the coating 27 can be configured such that the input impedance to each electrode 26 is measured at less than 13,000 ohms at 1 Hz.

The coating 27 can be a hydrogel that can be electrochemically grown or adhered to the electrode 26 when a current is passed through the electrode 26. In other examples, the coating 27 can be mechanically applied to each electrode 26 by spraying, painting, dipping, or otherwise covering the electrode 26 with the coating 27. The coating can have a thickness between 10 nanometers and 10 microns. In some examples, the coating can be a thickness that is less than the thickness of the covering 54 such that the covering 54 can help to protect the coating 27 from contacting the coupler 16, the deflectable element 12, or other objects which can damage the coating 27.

Reference is now made to Figs. 8B-8I, which illustrate example apertures 55 formed in a covering of the flexible polymer circuit strips of Fig. 7. By forming apertures 55 formed through the covering 54 to expose a surface of the electrode 26, the exposed surface of the electrode 26 can be coated with the coating 27. As illustrated in Figs. 8B-8I, the apertures 55 can be many shapes, sizes, and configurations. As will be appreciated by one of ordinary skill in the art, by changing the shape, size, and configuration of the apertures 55, the amount of exposed surface area of the electrodes 26 can be either increased or decreased, effectively increasing or decreasing the conductive surface area of the electrode 26. Furthermore, by increasing or decreasing the exposed surface area of the electrodes 26, the amount of coating 27 that can be applied to an aperture 55 will also be increased or decreased. In other words, as the apertures 55 increase in size, the surface area of the coating 27 in each aperture 55 will also increase which can cause the coating 27 to be more likely to rub on objects and become delaminated. Thus, as the aperture 55 size decreases, the covering 54 can provide more mechanical protection to the coating 27 to help reduce the likelihood of the coating 27 coming into contact with objects as the basket catheter 10 is used. As will be appreciated, however, as the size of a given aperture 55 is reduced, the conductive surface area of the electrode 26 will also be reduced. Therefore, the size, shape, and configuration of the apertures 55 over an electrode 26 can be optimized to allow for the electrode to sufficiently detect electrical signals while also ensuring the covering 54 provides sufficient mechanical protection to the coating 27. Manufacturability is another consideration. Presently, it is preferred that features of the covering 54 are at least 0.003 inches (76 microns) to avoid damaging the covering 54 between apertures 55 during manufacturing.

Fig. 8B illustrates an example electrode 26 of a flexible polymer circuit strip 24 having circular apertures 55A through the covering 54. In this example, eight circular apertures 55A can be formed through the covering 54 with each circular aperture 55A being spaced equally from each other. As will be appreciated, more or fewer circular aperture 55A can be formed through the covering 54 depending on the application. Furthermore, in some examples, the circular apertures 55A can be unequally spaced from each other. The coating 27 can be adhered to the exposed surface of the electrode 26 within each circular aperture 55A.

In other examples, the apertures 55 can comprise a polygonal shape. For example, Fig. 8C illustrates an electrode 26 of a flexible polymer circuit strip 24 having rectangular apertures 55B through the covering 54. In this example, fifteen rectangular apertures 55B can be formed through the covering 54 with each rectangular aperture 55B being spaced equally from each other. The rectangular apertures 55B can comprise a square or other rectangular shape. As another example, Fig. 8D illustrates an electrode 26 of a flexible polymer circuit strip 24 having decagonal apertures 55C through the covering 54. In this example, fifteen decagonal apertures 55C can be formed through the covering 54 with each decagonal aperture 55C being spaced equally from each other. As yet another example, Fig. 8E illustrates an electrode 26 of a flexible polymer circuit strip 24 having triangular apertures 55D through the covering 54. In this example, nineteen triangular apertures 55D can be formed through the covering 54. The triangular apertures 55D can be offset between each row of triangular apertures 55D such that a first row comprises four triangular apertures 55D while a second row comprises three triangular apertures 55D. Further, alternating rows can be inverted in relation to the one previous. This can allow for partially nesting of the tip of the inverted triangular aperture 55D between two other triangular apertures 55D in the previous row. The coating 27 can be adhered to the exposed surface of the electrode 26 within each rectangular aperture 55B, decagonal aperture 55C, triangular aperture 55D, etc.

As will be appreciated by one of skill in the art, apertures 55 of various other shapes and sizes can be formed through the covering 54 to expose the surface of the electrode 26. Furthermore, apertures 55 of various shapes can be formed through the covering 54 over a single electrode 26. For example, circular apertures 55A, decagonal apertures 55C, and triangular apertures 55D can be formed together over a single electrode 26. Similarly, apertures 55 of one size can be formed through the covering 54 over an electrode 26 along with apertures 55 of a different size. Further still, the apertures 55 may be equally spaced across the surface of the electrode 26 or unequally spaced across the surface of the electrode 26.

Figs. 8F and 8G illustrate example electrodes 26 of a flexible polymer circuit strip 24 having apertures 55 which are elongated slits 55E, 55F formed through the covering 54. At least four elongated slits 55E, 55F can be formed through the covering 54 to expose the surface of the electrode 26, although it will be appreciated that more or fewer elongated slits 55E, 55F can be formed depending on the application. In the example illustrated in Fig. 8F, the elongated slits 55E can extend from near one end of the electrode 26 to near a second end of the electrode 26 in a lengthwise direction. In the example illustrated in Fig. 8G, the elongated slits 55E can extend from near one end of the electrode 26 to near a second end of the electrode 26 in a widthwise direction.

As will be appreciated by one of skill in the art, by forming elongated slits 55E, 55F through the covering 54, a greater continuous surface area of the electrode 26 may be exposed which can help to increase the exposed conductive surface area of the electrode 26 but may also increase the likelihood of the coating 27 being rubbed while in use. Therefore, the spacing and size of the elongated slits 55E, 55F can be varied to help ensure the electrode 26 has a sufficient amount of surface area exposed while also ensuring the coating 27 is sufficiently protected.

Fig. 8H illustrates an example electrode 26 of a flexible polymer circuit strip 24 having apertures 55 which are elongated slits 55G formed through the covering 54. Unlike the elongated slits 55E, 55F illustrated in Figs. 8F and 8G, the elongated slits 55G extend only a portion of the length of the electrode 26 (e.g., approximately less than 1/3 of the length of the electrode 26). In this way, the elongated slits 55G can be configured to provide greater mechanical protection to the coating 27 but still ensure a sufficient amount of the electrode 26 is exposed.

Fig. 8I illustrates an example electrode 26 of a flexible polymer circuit strip 24 having a combination of circular apertures 55A and elongated slits 55E. In this example, the elongated slits 55E can help to increase the exposed surface area of the electrode 26 while the circular apertures 55A can expose some of the surface area of the electrode 26 while also helping to provide greater mechanical protection to the coating 27. As will be appreciated by one of skill in the art, any of the example apertures 55A-55D and elongated slits 55E-55G can be combined to help ensure a sufficient amount of the electrode 26 is exposed while also ensuring the coating 27 is suitably protected.

Reference is now made to Fig. 8J, which is a table (Table 1) illustrating impedance values for example patterns of apertures 55 formed in the covering of the flexible polymer circuit strips of Fig. 7. Although Table 1 illustrates impedance values that were experimentally obtained for a few selected patterns of apertures 55, impedance values may also be obtained for any of the patterns of apertures 55 described herein. Thus, Table 1 should not be construed as limiting but is offered to illustrate the impedance values of a few example patterns of apertures 55.

As illustrated in Fig. 8J, the impedance values (in ohms) for six different aperture 55 patterns and two control samples (one with coating 27 covering approximately 100% of the electrode 26 surface and one without any coating 27) are shown at frequencies of 1 Hz, 10 Hz, 50 Hz, and 100 Hz. As illustrated, as the input frequency increases, the impedance generally decreases. Furthermore, the impedance values are inversely related to the exposed surface area. Illustrations of the six different aperture 55 patterns are shown below Table 1 for explanatory purposes.

Starting from left to right in table 1, impedance data of a first example electrode 26 (Example 1) having three rows of seven circular apertures 55A in each row is shown. The impedance of Example 1 can range from approximately 10,406 ± 920 ohms at 1 Hz to approximately 168 ± 28 ohms at 100 Hz. Example 2 similarly illustrates an electrode 26 having circular apertures 55A, however, Example 2 comprises two rows of five circular apertures 55A in each row. As shown, the impedance of Example 2 can range from approximately 12,502 ± 552 ohms at 1 Hz to approximately 206 ± 20 ohms at 100 Hz. As will be appreciated, because Example 2 has less surface area of the electrode 26 coated with the coating 27, the covering 54 covers a greater amount of the surface area of the electrode 26 and can be more mechanically robust since more covering 54 material can be located between each circular aperture 55A.

Continuing from left to right in Table 1, Example 3 illustrates an electrode 26 having four elongated slits 55E stretching from near one end of the electrode 26 to near a second end of the electrode 26. The impedance of Example 3 can range from approximately 7,000 ± 467 ohm at 1 Hz to approximately 109 ± 4 ohms at 100 Hz. Example 4 illustrates an electrode 26 having three rows of elongated slits 55G with each elongated slit 55G extending only a portion of the electrode 26 surface. In particular, Example 4 comprises three rows of three elongated slits 55G. The impedance of Example 4 can range from approximately 10,544 ± 235 ohms at 1 Hz to approximately 164 ± 8 ohms at 100 Hz. As will be appreciated, because the elongated slits 55G of Example 4 extend only a portion of the surface of the electrode 26, the coating 27 can be more mechanically protected by the covering 54 when compared to Example 3.

Example 5 and Example 6 in Table 1 illustrate electrodes 26 having an aperture 55 sized to expose approximately one-third and two-thirds of the electrode 26 respectively. As shown, the impedance value of Example 5 can range from approximately 16,921± 4,158 ohms at 1 Hz to 306 ± 77 ohms at 100 Hz while the impedance value of Example 6 can range from approximately 9,951 ± 407 ohms at 1 Hz to 186 ± 24 ohms at 100 Hz. As will be appreciated, although the impedance may be reduced by having a larger aperture size 55 as shown in Example 6, the coating 27 may have a greater tendency of being damaged because the covering 54 is less able to provide mechanical protection to the coating 27.

In the two far right columns of Table 1, impedance values for two control examples are included for reference. First, a control showing an electrode 26 having approximately 100% of its surface coated with the coating 27 is shown. In this example, the overall impedance can range from approximately 6,629 ± 197 ohms at 1 Hz to 117 ± 3 ohms at 100 Hz. In the second control example, an electrode having none of its surface coated with the coating 27 is shown. The impedance values for an electrode 26 not having any coating 27 can range from approximately 265,513 ± 9,186 ohms at 1 Hz to 3,636 ± 182 ohms at 100 Hz. As these two control examples illustrate, the coating 27 can help to significantly reduce the overall impedance of the electrode 26. However, as previously explained, the coating 27 can become damaged and eventually delaminate if the coating 27 is impacted by components of the basket catheter 10 or other objects. Thus, by forming apertures 55 through the covering 54 and then coating the electrode's 26 surface with the coating 27, the disclosed technology can reduce the overall impedance while also helping to reduce the likelihood of damaging the coating 27.

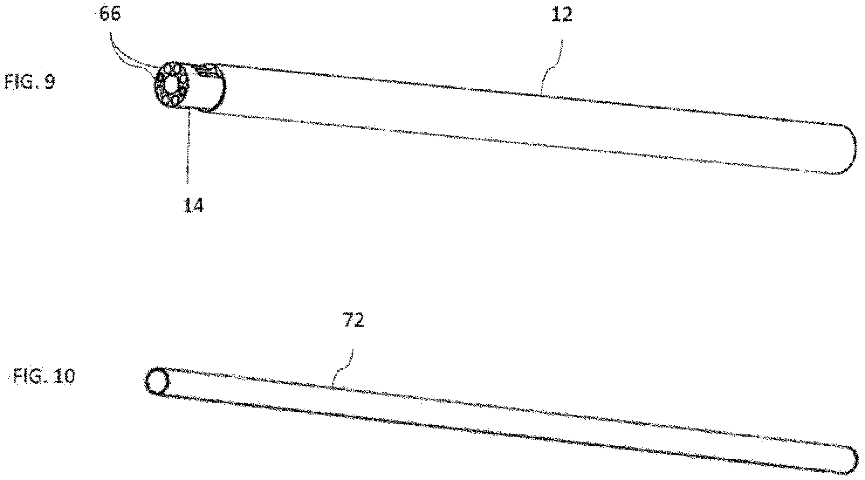

Reference is now made to Fig. 9, which is a schematic view of the elongated deflectable element 12 of the basket catheter 10 of Fig. 1. The elongated deflectable element 12 may be produced from any suitable material, for example, polyurethane or polyether block amide. The distal end 14 of the elongated deflectable element 12 has a smaller outer diameter than the rest of the elongated deflectable element 12 to accept the coupler 16 thereon as shown in Fig. 20. The elongated deflectable element 12 includes lumens 66 for inserting various tubes and wires therein as described herein. The elongated deflectable element 12 may have any suitable outer diameter and length, for example, the outer diameter may be in a range between 1 mm and 4 mm and the length may be in a range between 1 cm and 15 cm.

Reference is now made to Fig. 10, which is a schematic view of an irrigation sleeve 72 of the basket catheter 10 of Fig. 1. The irrigation sleeve 72 is a flexible tube which is disposed in one of the lumens 66 (Fig. 9) of the elongated deflectable element 12 (Fig. 9). The irrigation sleeve 72 may be used to carry irrigation fluid to the region of the expandable assembly 22 (Fig. 1). The irrigation sleeve 72 is sized to fit in one of the lumens 66 (typically a central lumen) of the elongated deflectable element 12 and extend beyond the distal end 14 (Fig. 9) of the elongated deflectable element 12 as shown in Fig. 20. The inner and outer diameter of the irrigation sleeve 72 may be in the range between 3mm and 5mm. The irrigation sleeve 72 may be formed from any suitable material, for example, but not limited to polyimide, polyurethane, polyether block amide, or polyethylene terephthalate.

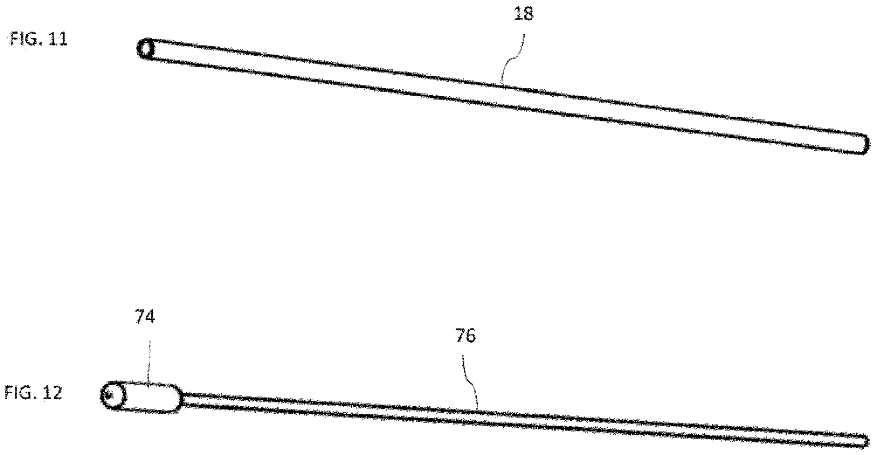

Reference is now made to Fig. 11, which is a schematic view of the pusher 18 of the basket catheter 10 of Fig. 1. The pusher 18 is a flexible tube and is disposed in the irrigation sleeve 72. The pusher 18 is sized to slide in the irrigation sleeve 72 and allow room for irrigation fluid to pass between the irrigation sleeve 72 and the pusher 18. The inner diameter of the pusher 18 is sized to accommodate wiring of a multi-axis position sensor described with reference to Fig. 12. The pusher 18 extends beyond the distal end 14 of the elongated deflectable element 12 (Fig. 9) until the nose connector 30 as shown in Fig. 19. The pusher 18 may be formed from any suitable material, for example, but not limited to polyimide with or without braiding, polyether ether ketone (PEEK) with or without braiding, or polyamide with or without braiding.

Reference is now made to Fig. 12, which is a schematic view of a multi-axis position sensor 74 of the basket catheter 10 of Fig. 1. The multi-axis position sensor 74 may comprise a dual-axis or triple-axis position sensor, for example, a magnetic position sensor comprising multiple orthogonal coils. Wiring 76 is used to connect the multi-axis position sensor 74 via the hollow of the pusher 18 (Fig. 11) to a position computation system (not shown) disposed proximally to the basket catheter 10. The multi-axis position sensor 74 and the wiring 76 are shown in more detail in Figs. 5 and 19.

Reference is now made to Figs. 13A-B, which are schematic views of the nose connector 30 of the basket catheter 10 of Fig. 1. The nose connector 30 may be formed from any suitable material, for example, but not limited to polycarbonate with or without glass filler, PEEK with or without glass filler, or PEI with or without glass filler. The nose connector 30 includes a proximal cavity 78(Fig. 13A) in which the pusher 18 (Fig. 11) is secured and through which the wiring 76 passes as shown in Fig. 19. Fig. 13B also shows the distal receptacle 34, the inner surface 36, and the distal facing opening 38. The distal receptacle 34 houses the multi-axis position sensor 74 (Fig. 12) and the hinges 28 (Fig. 5) which are connected to the inner surface 36.

Reference is now made to Fig. 14, which is a schematic view of the center electrode ring 40 of the basket catheter 10 of Fig. 1. Electrode 40 is electrically connected to a wire (not shown) that passes through the slot in the side of proximal cavity 78 and into pusher 18. The center electrode ring 40 may be formed from any suitable material, for example, but not limited to noble metals and their alloys comprising platinum, palladium, gold, or iridium. The center electrode ring 40 serves a secondary role by providing mechanical support around the proximal cavity 78 (Fig. 13A) of the nose connector 30 to secure the nose connector 30 to the pusher 18 (Fig. 11) as shown in Fig. 19.

Reference is now made to Figs. 15A-B, which are schematic views of the nose cap 32 of the basket catheter 10 of Fig 1. The nose cap 32 includes a hollow cylinder 80 covered with a cover 82 which may be wider than the hollow cylinder 80. The nose cap 32 may be formed from any suitable material, for example, but not limited to polycarbonate with or without glass filler, PEEK with or without glass filler, or PEI with or without glass filler. The nose cap 32 is sized to fit in the distal receptacle 34 (Fig. 13B) of the nose connector 30 (Fig. 13B) and cover the distal facing opening 38 (Fig. 13B) while allowing space for the multi-axis position sensor 74 (Fig. 12) and the hinges 28 (Fig. 5) therein as shown in Fig. 19. The nose cap 32 may optionally be sized to provide a pressure fit against the hinges 28 to prevent the hinges 28 from being pulled away from the inner surface 36 (Fig. 13B) of the nose connector 30 (Fig. 13B). The nose connector 30 may also function to protect the multi-axis position sensor 74.

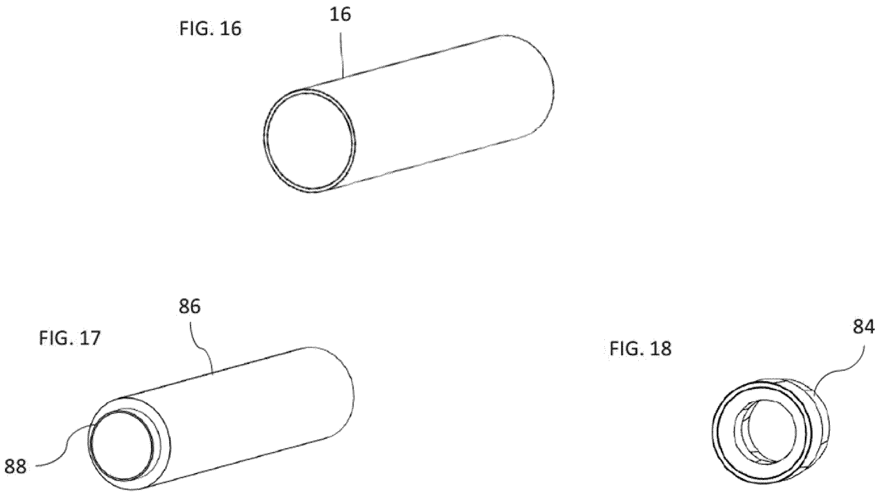

Reference is now made to Fig. 16, which is a schematic view of the coupler 16 of the basket catheter 10 of Fig. 1. The coupler 16 typically comprises a hollow tube and may be formed from any suitable material, for example, but not limited to polycarbonate with or without glass filler, PEEK with or without glass filler, polyimide, polyamide, or PEI with or without glass filler. The coupler 16 may be sized to have the same inner diameter as the outer diameter of the distal end 14 (Fig. 9) of the elongated deflectable element 12 (Fig. 9) and the same outer diameter as the proximal portion of the elongated deflectable element 12. The coupler 16 is also sized to surround various elements described in more detail with reference to Fig. 20.

Reference is now made to Fig. 17, which is a schematic view of a single-axis position sensor 86 of the basket catheter 10 of Fig. 1. The single-axis position sensor 86 may include any suitable position sensor, for example, a magnetic position sensor comprising a coil wound on a hollow cylinder 88. Wiring (not shown) from the single-axis position sensor 86 may be passed down one of the lumens 66 (Fig. 9) to a position computation system (not shown) disposed proximally to the basket catheter 10. The hollow cylinder 88 is sized to accommodate the irrigation sleeve 72 therein as shown in Fig. 20. The outer diameter and length of the single-axis position sensor 86 is sized to fit in the coupler 16 (Fig. 16). The hollow cylinder 88 may be formed from any suitable material, for example, but not limited to, a material used as a magnetic core.

Reference is now made to Fig. 18, which is a schematic view of a proximal retainer ring 84 of the basket container 10 of Fig. 1. The proximal retainer ring 84 is configured to provide a pressure fit around the distal end of the irrigation sleeve 72 (Fig. 10) and retain the single-axis position sensor 86 (Fig. 17) to be adjacent to the distal end 14 (Fig. 9) of the elongated deflectable element 12 (Fig. 9) as shown in Fig. 20. The proximal retainer ring 84 also serves to secure the flexible polymer circuits 24 between the retainer ring 84 and the coupler 16. The proximal retainer ring 84 may be formed from any suitable material, for example, but not limited to polycarbonate with or without glass filler, PEEK with or without glass filler, or PEI with or without glass filler.

Reference is now made to Figs. 19-20, which are cross sectional views through line A-A of Fig. 1. Fig. 19 shows a distal portion of the expandable assembly 22, while Fig. 20 shows a proximal portion.

Fig. 19 shows that the distal portion 20 of the pusher 18 is disposed in the proximal cavity 78 of the nose connector 30 and is secured therein using the center electrode ring 40 disposed around the outside of the proximal cavity 78. The multi-axis position sensor 74 is disposed in the distal receptacle 34 of the nose connector 30 with the wiring 76 extending proximally through the pusher 18. The second ends 46 of the flexible polymer circuit strips 24 are connected to the inner surface 36 of the distal receptacle 34 of the nose connector 30. The elongated resilient support elements 48 extend along the length of the flexible polymer circuit strips 24 until, but not including, the hinges 28. The nose cap 32 is inserted into the distal receptacle 34 with the hollow cylinder 80 surrounding the distal portion of the multi-axis position sensor 74 and providing pressure against the second ends 46 of the flexible polymer circuit strips 24. The nose cap 32 covers the distal facing opening 38 of the nose connector 30.

Fig. 20 shows that the irrigation sleeve 72 is disposed in the elongated deflectable element 12. The pusher 18 is disposed in the irrigation sleeve 72. The wiring 76 is disposed in the pusher 18. The single-axis position sensor 86 is disposed around the irrigation sleeve 72 (between the coupler 16 and the pusher 18) close to the distal end 14 of the elongated deflectable element 12. The proximal retainer ring 84 provides a pressure fit around the irrigation sleeve 72 and keeps the single-axis position sensor 86 in place distally to the distal end 14 of the elongated deflectable element 12. The proximal end of the coupler 16 is connected to the distal end 14 of the elongated deflectable element 12. The first ends 42 of the flexible polymer circuit strips 24 are connected to the inner surface 44 of the coupler 16. Fig. 20 shows that the elongated resilient support elements 48 extend along the respective strips 24 from the coupler 16 until before the respective hinges 28 (Fig. 19).

While the expandable assembly is shown without being mounted to a flexible membrane, it is possible that the expandable assembly can be provided with a membrane (e.g., balloon like surface) as a base substrate for the circuit strips. As well, the membrane can be used as a covering layer over the circuit strips 24 with electrodes 26 being exposed (or not covered by the membrane for exposure) to the ambient environment (e.g., inside organ tissues).

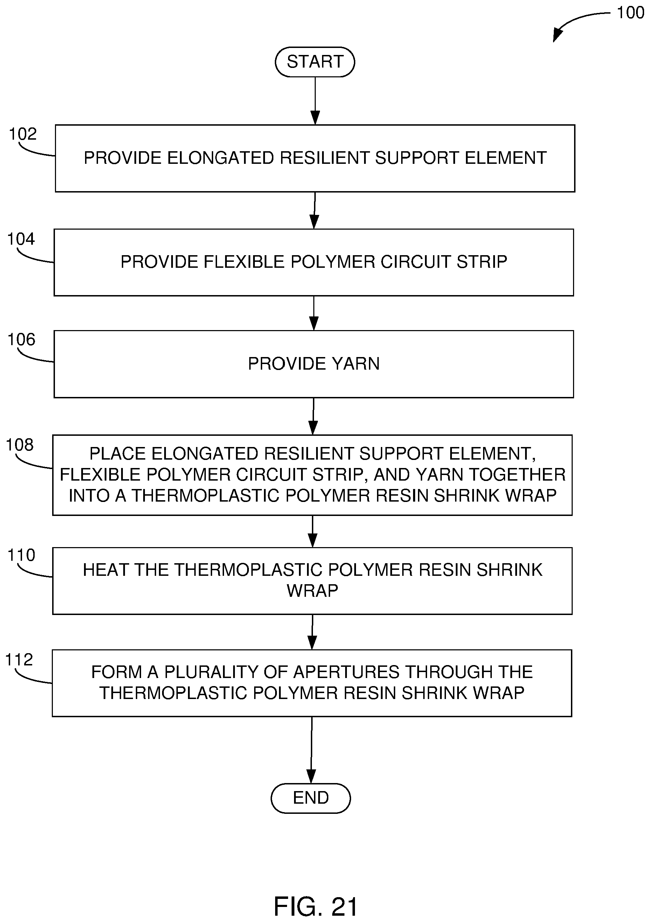

Reference is now made to Fig. 21, which illustrates an example method 100 of manufacturing a flexible polymer circuit strip 24 as described herein. The method 100 can include providing 102 an elongated resilient support element (e.g., elongated resilient support element 48), providing 104 a flexible polymer circuit (e.g., flexible polymer circuit strip 24), and providing 106 a yarn (e.g., yarn 52). The method can further include placing 108 the elongated resilient support element, the flexible polymer circuit, and the yarn together into a thermo plastic polymer resin shrink wrap (PET) (e.g., covering 54). The PET can then be heated 110 to shrink the PET around the elongated resilient support element, the flexible polymer circuit, and the yarn to at least partially enclose the elongated resilient support element, the flexible polymer circuit, and the yarn.

The method 100 can further include forming 112 a plurality of apertures through the PET to expose the surface of each electrode (e.g., electrode 26) on the flexible polymer circuit. As will be appreciated by one of ordinary skill in the art with the benefit of this disclosure, forming 112 the plurality of apertures through the PET can include any of the examples shown and described in this disclosure. For example, forming 112 the plurality of apertures through the PET can include forming circular apertures 55A as shown and described in relation to Fig. 8B. As another example, forming 112 the plurality of apertures through the PET can include forming elongated strips 55E as shown and described in relation to Fig. 8F. Forming 112 the plurality of apertures through the PET can further include any combination of the examples shown and described in this disclosure and any of the methods described herein.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable subcombination. The embodiments described above are cited by way of example, and the present invention is not limited by what has been particularly shown and described hereinabove.

## Claims

1. A catheter apparatus (10), comprising:
an elongated deflectable element (12) including a distal end;
a coupler (16) connected to the distal end (14);
a pusher (18) including a distal portion (20), and being configured to be advanced and retracted through the deflectable element (12); and
an expandable assembly (22) comprising a plurality of flexible polymer circuit strips (24), with first ends of the flexible polymer circuit strips (24) being connected to the coupler (16) and second ends of the flexible polymer circuit strips (24) being connected to the distal portion of the pusher (18), the flexible polymer circuit strips (24) being configured to bow radially outward when the pusher (18) is retracted expanding the expandable assembly (22) from a collapsed form to an expanded form, each flexible polymer circuit strip (24) comprising:
multiple electrodes (26) disposed thereon, each electrode (26) defining a conductive surface;
a covering (54) partially enclosing the flexible polymer circuit strip (24) and the multiple electrodes (26), the covering comprising a plurality of apertures (55) at each electrode of the multiple electrodes (26) so that a portion of the conductive surface of each electrode (26) is exposed through each aperture (55) of the plurality of apertures (55); and **characterised by**
a conductive polymer coating disposed in each aperture of the plurality of apertures configured to provide an input impedance to each electrode (26) of less than 13,000 ohms at 1 Hz.

2. The apparatus (10) according to claim 1, the covering comprising a non-conductive polymer material and the conductive surface of each electrode (26) is disposed approximately 12 microns below an outer surface of the covering.

3. The apparatus (10) according to claim 1, the plurality of apertures comprising a plurality of circular apertures at each electrode (26) of the multiple electrodes (26).

4. The apparatus (10) according to claim 1, the plurality of apertures comprising a plurality of polygonal apertures at each electrode (26) of the multiple electrodes (26).

5. The apparatus (10) according to claim 4, the plurality of polygonal apertures comprising rectangular apertures or decagonal apertures.

6. The apparatus (10) according to claim 1, the plurality of apertures comprising a plurality of elongated slits at each electrode of the multiple electrodes, each elongated slit of the plurality of elongated slits extending from near a first end of the electrode to near a second end of the electrode.

7. The apparatus (10) of claim 1, wherein the flexible polymer circuit strips further comprise:
an elongated resilient support element;
a flexible polymer circuit connected to the elongated resilient support element, the flexible polymer circuit comprising the plurality of electrodes, each electrode defining a first conductive surface area; and
wherein the covering at least partially encloses the elongated resilient support element, the flexible polymer circuit and the plurality of electrodes, wherein the apertures over each electrode collectively defines a second conductive surface area of approximately less than half of the first conductive surface area.

8. The apparatus (10) of claim 7, the plurality of apertures comprising a plurality of circular apertures at each electrode of the plurality of electrodes.

9. The apparatus (10) of claim 7, the plurality of apertures comprising a plurality of polygonal apertures at each electrode of the plurality of electrodes.

10. The apparatus (10) of claim 9, the plurality of polygonal apertures comprising rectangular apertures.

11. The apparatus (10) of claim 7, the plurality apertures comprising a plurality of elongated slits at each electrode of the plurality of electrodes, each elongated slit of the plurality of elongated slits extending from near a first end of the electrode to near a second end of the electrode.

12. The apparatus according to claim 7, the elongated resilient support element comprising Nitinol or Polyetherimide (PEI).

13. The apparatus of claim 1, in which an impedance measured from the electrodes comprise less than 1400 ohms at 10 Hz, approximately 300 ohms or less at 50 Hz, and approximately 200 ohms or less at 100 Hz.

14. The apparatus of claim 13, in which the plurality of apertures comprises two rows of five substantially circular apertures per row or three rows of seven substantially circular apertures per row.

## Patentansprüche

1. Kathetereinrichtung (10), umfassend:
ein längliches, ablenkbares Element (12), das ein distales Ende einschließt;
eine Kupplung (16), die mit dem distalen Ende (14) verbunden ist;
einen Drücker (18), der einen distalen Abschnitt (20) einschließt und konfiguriert ist, um durch das ablenkbare Element (12) vorwärts und rückwärts bewegt werden zu können; und
eine expandierbare Anordnung (22), umfassend eine Vielzahl von flexiblen Polymer-Leiterstreifen (24), wobei erste Enden der flexiblen Polymer-Leiterstreifen (24) mit der Kupplung (16) verbunden sind und zweite Enden der flexiblen Polymer-Leiterstreifen (24) mit dem distalen Abschnitt des Drückers (18) verbunden sind, wobei die flexiblen Polymer-Leiterstreifen (24) konfiguriert sind, um sich radial nach außen wölben, wenn der Drücker (18) zurückgezogen wird, wodurch die expandierbare Anordnung (22) von einer kollabierten Form in eine expandierte Form expandiert wird, wobei jeder flexible Polymer-Leiterstreifen (24) umfasst:
mehrere darauf angeordnete Elektroden (26), wobei jede Elektrode (26) eine leitfähige Oberfläche definiert;
eine Abdeckung (54), die den flexiblen Polymer-Leiterstreifen (24) und die mehreren Elektroden (26) teilweise umschließt, die Abdeckung umfassend eine Vielzahl von Öffnungen (55) an jeder Elektrode der mehreren Elektroden (26), sodass ein Abschnitt der leitfähigen Oberfläche jeder Elektrode (26) durch jede Öffnung (55) der Vielzahl von Öffnungen (55) freigelegt ist; und **gekennzeichnet durch**
eine leitfähige Polymerbeschichtung, die in jeder Öffnung der Vielzahl von Öffnungen angeordnet und konfiguriert ist, um jeder Elektrode (26) eine Eingangsimpedanz von weniger als 13.000 Ohm bei 1 Hz bereitzustellen.

2. Einrichtung (10) nach Anspruch 1, wobei die Abdeckung ein nicht-leitfähiges Polymermaterial umfasst und die leitfähige Oberfläche jeder Elektrode (26) etwa 12 Mikrometer unterhalb einer Außenoberfläche der Abdeckung angeordnet ist.

3. Einrichtung (10) nach Anspruch 1, wobei die Vielzahl von Öffnungen eine Vielzahl von kreisförmigen Öffnungen an jeder Elektrode (26) der mehreren Elektroden (26) umfasst.

4. Einrichtung (10) nach Anspruch 1, wobei die Vielzahl von Öffnungen eine Vielzahl von polygonalen Öffnungen an jeder Elektrode (26) der mehreren Elektroden (26) umfasst.

5. Einrichtung (10) nach Anspruch 4, wobei die Vielzahl von polygonalen Öffnungen rechteckige Öffnungen oder zehneckige Öffnungen umfasst.

6. Einrichtung (10) nach Anspruch 1, wobei die Vielzahl von Öffnungen eine Vielzahl von länglichen Schlitzen an jeder der mehreren Elektroden umfasst, wobei sich jeder längliche Schlitz der Vielzahl von länglichen Schlitzen von nahe einem ersten Ende der Elektrode bis nahe einem zweiten Ende der Elektrode erstreckt.

7. Einrichtung (10) nach Anspruch 1, wobei die flexiblen Polymer-Leiterstreifen ferner umfassen:
ein längliches elastisches Stützelement;
eine flexible Polymerschaltung, die mit dem länglichen, elastischen Stützelement verbunden ist, wobei die flexible Polymerschaltung die Vielzahl von Elektroden umfasst, wobei jede Elektrode eine erste leitfähige Oberfläche definiert; und
wobei die Abdeckung das längliche elastische Stützelement, die flexible Polymerschaltung und die Vielzahl von Elektroden mindestens teilweise umschließt, wobei die Öffnungen über jeder Elektrode zusammen eine zweite leitfähige Oberfläche bilden, deren Fläche etwa weniger als die Hälfte der ersten leitfähigen Oberfläche beträgt.

8. Einrichtung (10) nach Anspruch 7, wobei die Vielzahl von Öffnungen eine Vielzahl von kreisförmigen Öffnungen an jeder Elektrode der Vielzahl von Elektroden umfasst.

9. Einrichtung (10) nach Anspruch 7, wobei die Vielzahl von Öffnungen eine Vielzahl von polygonalen Öffnungen an jeder Elektrode der Vielzahl von Elektroden umfasst.

10. Einrichtung (10) nach Anspruch 9, wobei die Vielzahl von polygonalen Öffnungen rechteckige Öffnungen umfasst.

11. Einrichtung (10) nach Anspruch 7, wobei die Vielzahl von Öffnungen eine Vielzahl von länglichen Schlitzen an jeder der Vielzahl von Elektroden umfasst, wobei sich jeder längliche Schlitz der Vielzahl von länglichen Schlitzen von nahe einem ersten Ende der Elektrode bis nahe einem zweiten Ende der Elektrode erstreckt.

12. Einrichtung nach Anspruch 7, die länglichen, elastischen Stützelemente umfassend Nitinol oder Polyetherimid (PEI).

13. Einrichtung nach Anspruch 1, wobei eine an den Elektroden gemessene Impedanz weniger als 1400 Ohm bei 10 Hz, etwa 300 Ohm oder weniger bei 50 Hz und etwa 200 Ohm oder weniger bei 100 Hz umfasst.

14. Einrichtung nach Anspruch 13, wobei die Vielzahl von Öffnungen zwei Reihen von fünf im Wesentlichen kreisförmigen Öffnungen pro Reihe oder drei Reihen von sieben im Wesentlichen kreisförmigen Öffnungen pro Reihe umfasst.

## Revendications

1. Appareil de cathéter (10), comprenant :
un élément pouvant fléchir (12) allongé comportant une extrémité distale ;
un dispositif d'accouplement (16) relié à l'extrémité distale (14) ;
un dispositif de poussée (18) comportant une partie distale (20), et conçu pour être avancé et rétracté à travers l'élément pouvant fléchir (12) ; et
un ensemble extensible (22) comprenant une pluralité de bandes de circuit en polymère flexible (24), des premières extrémités des bandes de circuit en polymère flexible (24) étant reliées au dispositif d'accouplement (16) et des secondes extrémités des bandes de circuit en polymère flexible (24) étant reliées à la partie distale du dispositif de poussée (18), les bandes de circuit en polymère flexible (24) étant conçues pour se courber radialement vers l'extérieur lorsque le dispositif de poussée (18) est rétracté, étendant l'ensemble extensible (22) d'une forme repliée à une forme étendue, chaque bande de circuit en polymère flexible (24) comprenant :
plusieurs électrodes (26) disposées sur celle-ci, chaque électrode (26) définissant une surface conductrice ;
un revêtement (54) recouvrant partiellement la bande de circuit en polymère flexible (24) et les électrodes (26), le revêtement comprenant une pluralité d'ouvertures (55) au niveau de chaque électrode parmi les électrodes (26) de sorte qu'une partie de la surface conductrice de chaque électrode (26) est exposée à travers chaque ouverture (55) de la pluralité d'ouvertures (55) ; et **caractérisé par**
un revêtement polymère conducteur disposé dans chaque ouverture parmi la pluralité d'ouvertures, conçu pour fournir à chaque électrode (26) une impédance d'entrée allant de moins de 13 000 ohms à 1 Hz.

2. Appareil (10) selon la revendication 1, le revêtement comprenant un matériau polymère non conducteur et la surface conductrice de chaque électrode (26) est disposée à environ 12 microns sous une surface extérieure du revêtement.

3. Appareil (10) selon la revendication 1, la pluralité d'ouvertures comprenant une pluralité d'ouvertures circulaires au niveau de chaque électrode (26) parmi les électrodes (26).

4. Appareil (10) selon la revendication 1, la pluralité d'ouvertures comprenant une pluralité d'ouvertures polygonales au niveau de chaque électrode (26) parmi les électrodes (26).

5. Appareil (10) selon la revendication 4, la pluralité d'ouvertures polygonales comprenant des ouvertures rectangulaires ou des ouvertures décagonales.

6. Appareil (10) selon la revendication 1, la pluralité d'ouvertures comprenant une pluralité de fentes allongées au niveau de chaque électrode parmi les électrodes, chaque fente allongée parmi la pluralité de fentes allongées s'étendant depuis près d'une première extrémité de l'électrode jusqu'à près d'une seconde extrémité de l'électrode.

7. Appareil (10) selon la revendication 1, dans lequel les bandes de circuit en polymère flexible comprennent en outre :
un élément de support allongé et souple ;
un circuit en polymère flexible relié à l'élément de support allongé et souple, le circuit en polymère flexible comprenant la pluralité d'électrodes, chaque électrode définissant une première zone de surface conductrice ; et
dans lequel le revêtement entoure au moins partiellement l'élément de support allongé et souple, le circuit en polymère flexible et la pluralité d'électrodes, dans lequel les ouvertures au-dessus de chaque électrode définissent collectivement une seconde zone de surface conductrice d'environ moins de la moitié de la première zone de surface conductrice.

8. Appareil (10) selon la revendication 7, la pluralité d'ouvertures comprenant une pluralité d'ouvertures circulaires au niveau de chaque électrode parmi la pluralité d'électrodes.

9. Appareil (10) selon la revendication 7, la pluralité d'ouvertures comprenant une pluralité d'ouvertures polygonales au niveau de chaque électrode parmi la pluralité d'électrodes.

10. Appareil (10) selon la revendication 9, la pluralité d'ouvertures polygonales comprenant des ouvertures rectangulaires.

11. Appareil (10) selon la revendication 7, la pluralité d'ouvertures comprenant une pluralité de fentes allongées au niveau de chaque électrode parmi la pluralité d'électrodes, chaque fente allongée parmi la pluralité de fentes allongées s'étendant depuis près d'une première extrémité de l'électrode jusqu'à près d'une seconde extrémité de l'électrode.

12. Appareil selon la revendication 7, l'élément de support allongé et souple comprenant du Nitinol ou du polyétherimide (PEI).

13. Appareil selon la revendication 1, dans lequel une impédance mesurée à partir des électrodes comprend moins de 1400 ohms à 10 Hz, environ 300 ohms ou moins à 50 Hz, et environ 200 ohms ou moins à 100 Hz.

14. Appareil selon la revendication 13, dans lequel la pluralité d'ouvertures comprend deux rangées de cinq ouvertures sensiblement circulaires par rangée ou trois rangées de sept ouvertures sensiblement circulaires par rangée.
